Europäisches Patentamt

European Patent Office

Office européen des brevets

(11) Publication number: **0 239 256**
**A1**

# (12) EUROPEAN PATENT APPLICATION

(21) Application number: **87301727.1**

(22) Date of filing: **26.02.87**

(51) Int. Cl.⁴: **A61M 1/36**

(30) Priority: **27.02.86 US 834292**

(43) Date of publication of application:
**30.09.87 Bulletin 87/40**

(84) Designated Contracting States:
**AT BE DE ES FR GB IT SE**

(71) Applicant: **McNeilab, Inc.**

**Springhouse Pennsylvania 19477(US)**

(72) Inventor: **King, Martin J.**
**12400 91st Avenue North**
**Seminole Florida 33542(US)**
Inventor: **Troutner, Vernon H.**
**6221 58th Avenue North**
**St. Petersburg Florida 33709(US)**
Inventor: **Goss, Jack**
**1717 Winfield Road**
**Clearwater Florida 33516(US)**

(74) Representative: **Jones, Alan John et al**
**CARPMAELS & RANSFORD 43 Bloomsbury Square**
**London, WC1A 2RA(GB)**

(54) **Concurrent on-line irradiation treatment system.**

(57) In a system for altering cells such as by irradiating said cells when in contact with a photoactivatable reagent such as for the extracorporeal treatment of a patient's blood, a concurrent on-line method for performing the operations of collection, enrichment, irradiation and return in a safe and efficacious manner.

FIG-1

## CONCURRENT ON-LINE IRRADIATION TREATMENT SYSTEM

This invention relates to the field of treating cells with photoactivatable compounds and radiation which activates the compound thereby affecting the cells and specifically, relates to clinically useful systems for the extracorporeal treatment of blood cells, especially leukocytes, with UV radiation, and more particularly to methods for concurrently collecting, separating, and irradiating blood cells while keeping the patient on-line.

It is well-known that a number of human disease states may be characterized by the overproduction of certain types of leukocytes, including lymphocytes, in comparison to other populations of cells which normally comprise whole blood. Excessive or abnormal lymphocyte populations result in numerous adverse effects to patients including the functional impairment of bodily organs, leukocyte mediated autoimmune diseases and leukemia related disorders many of which often ultimately result in fatality.

U.S. Patent Nos. 4,321,919; 4,398,906; 4,428,744; and 4,464,166 to Edelson describe methods for treating blood whereby the operation or viability of certain cellular populations may be moderated thereby providing relief for these patients. In general, the methods comprise treating the blood with a dissolved photoactivatable drug, such as psoralen, which is capable of forming photoadducts with DNA in the presence of U.V. radiation. It is believed that covalent bonding results between the psoralen and the lymphocyte nucleic acid thereby effecting metabolic inhibition of the thusly treated cells. Following extracorporeal radiation, the cells are returned to the patient where they are thought to be cleared by natural processes but at an accelerated pace believed attributable to disruption of membrane integrity, alteration of DNA within the cell, or the like conditions often associated with substantial loss of cellular effectiveness or viability.

Although a number of photoactivatable compounds in the psoralen class are known, 8-methoxy psoralen is presently the compound of choice. An effective radiation for this compound, and many psoralens in general, is the ultraviolet spectrum in the range of approximately 320 to 400 nanometers, alternatively referred to as the U.V.A. spectrum. As the development of photoactivatable compounds proceeds, it may be expected that changes in the preferred activation radiation spectrum will be necessary. Suitable selection of radiation sources will, of course, increase treatment efficiency and is contemplated as an obvious optimization procedure for use with the inventions disclosed herein.

Although Edelson's methods have been experimentally shown to provide great relief to patients suffering from leukocyte mediated diseases, numerous practical problems require solutions. In particular, Edelson fails to provide a suitable apparatus for applying radiation to the cells, e.g. via a treatment station, in an economical and efficacious manner, or a system for incorporating a treatment station providing for the treatment of a patient in a clinically acceptable format.

Conventional techniques for photoactivating compounds associated with cells have relied on a plurality of devices including flasks, filtration columns, spectrophotometer cuvettes, and petri dishes. The sample to be irradiated is added to the containers and the container placed adjacent to the radiation source. Such systems tend to be laboratory curiosities as they fail to provide the necessary safeguards intrinsically necessary where patient bodily fluids are concerned, particularly since these fluids must be returned to the patient thereby necessitating strict avoidance of contamination. Further, such methods tend to be volume limited, are characterized by many mechanical manipulations and are generally unacceptable from a clinical and regulatory viewpoint. It is an object of the present invention to provide methods and apparatus suitable for use with the Edelson methods to overcome the limitations associated with the conventional expedients.

EP-A-0 138 489 describes a practical device for coupling the radiation provided by commercially available light sources, such as the so-called "black-light" fluorescent tubes, to cells for treatment by Edelson's photoactivated drug methods. In summary, the disposable cassette described therein comprises a plurality of fluorescent tube-like light sources such as the U.V.A. emitting Sylvania F8TS/BLB bulb, which are individually, coaxially mounted in tubes of larger diameter which are, in turn, coaxially mounted in sealing arrangement within second outer tubes of even larger diameter thereby forming a structure having two generally elongated, cylindrical cavities about each radiation source. The inner cavity preferably communicates with the atmosphere thereby facilitating cooling of the radiation source. The second tube forming the outer cavity further comprises inlet and outlet means for receiving and discharging, respectively, the cells to be irradiated. A plurality of these structures are "ganged" and suitable connections made between inlets and outlets of adjacent members to provide for serpentine flow of cells through each outer cavity. Thus, continuous flow of the cells through the plurality of cavities surrounding the centrally disposed radiation sources facilitates thorough treatment of the cells. Additional, detailed description of this device may be obtained by direct reference to EP-A-0 138 489.

To be fully practical, the Taylor device requires a clinically acceptable instrument to house the device and to provide the cells to be treated in an appropriate form. Such an instrument is the object of the inventions described in U.S. Patent Nos. 4,573,960 4,568,328 4,578,056, 4,573,961, 4,596,547, 4,623,328, and 4,573,962      While the instruments described therein work well, it is an object of the instant application to describe improved systems capable of implementing, in advanced fashion, the medical treatment principles first taught by Edelson.

It is another object of the present invention to provide still further improvements in greater patient safety and comfort while reducing treatment time and cost, by utilizing a newly designed disposable irradiation chamber in an appropriate instrument which incorporates a U.V.A. light array, more fully described in copending European European Nos.      and      respectively, claiming priority from USSN 834 258 and 834 256, respectively and having reference numbers JJC I4 and JJC 6 respectively.

It is yet another object to provide an improved instrument which meets the above criteria while maintaining the positive attributes of the prior system; compactness, mobility, completeness, fully automated and monitored, coupled with ease of operation.

It is a. further related object of this invention to provide, in contrast to the time consuming batch like processing of the prior system, continuous on-line patient treatment wherein collection, separation, and cell treatment occur simultaneously, thereby reducing treatment time and increasing patient safety and comfort.

These and still other objects of the invention will become apparant upon study of the accompanying drawings wherein:

Figure I illustrates a preferred configuration of the system during collection, separation, and treatment;

Figure series 2 shows the control panel for the preferred embodiment;

Figure series 3 shows a side view of a preferred embodiment of the irradiation chamber interfacing with various components of the preferred instrument; and

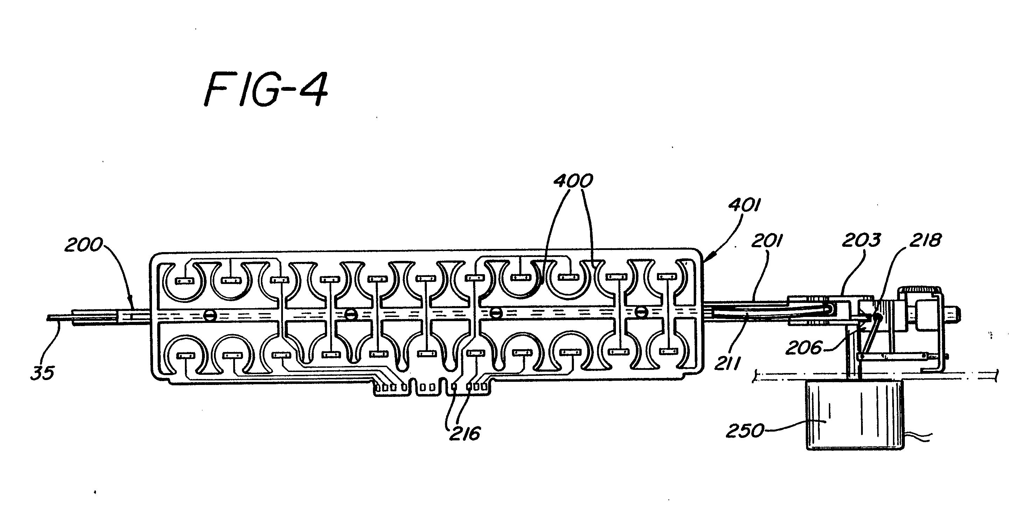

Figure 4 illustrates in a bottom view a preferred embodiment of the disposable light array for irradiating the irradiation chamber.

In accordance with the principles and objects of the present invention there are provided apparatus for "on-line" extracorporeally photoactivating a photoactivatable reagent in contact with blood cells by simultaneously performing the steps of collecting and separating on a continuous basis, blood from a patient while the patient is connected to the apparatus, returning undesired blood portions obtained during separation while the desired portion is photoactivatably treated and thereafter returning the thusly treated cells to the patient. As a result of this novel approach, the treatment system of the instant inventions optimizes and minimizes treatment time by concurrently conducting various aspects of such photoactivation treatment which were previously performed sequentially. More specifically, the apparatus collects and separates blood on a continuous basis as it is withdrawn from the patient and returns unwanted portions to the patient while concurrently energizing the irradiation sources for photoactivating the photoactivatable reagent in contact with the desired blood portion. Following photoactivation, the treated cells may then be facilely returned to the patient utilizing a drip chamber gravity feed infusion line incorporated in the tubing set. As may be readily appreciated, such procedures required novel apparatus to perform such operations while avoiding risk of harm to the patient.

Figure I shows various aspects of the system developed for extracorporeally treating a patient based in part upon the scientific discoveries of Edelson. The specific design, construction and operation of the apparatus 10 is the result of a number of separate invention. Some of these form the subject matter of previously described issued patent and published European applications

Some form the subject matter of the following concurrently filed European patent applications:

| Application Number | Publication Number | US Priority Application Number | Applicants Reference |
|---|---|---|---|
| | | 834 293 | JJC 3 |
| | | 834 294 | JJC 4 |
| | | 834 303 | JJC 5 |
| | | 834 256 | JJC 6 |
| | | 834 257 | JJC 7 |
| | | 834 260 | JJC 8 |
| | | 834 259 | JJC 9 |
| | | 834 258 | JJC 14 |

A brief description of the contents of these applications is included herein.

The operation of the device and performance of the methods can be divided into two basic phases or modes, depicted in part by Figure I. The first phase is shown substantially in Figure I wherein the patient is connected at the point shown, preferably by venipuncture or the like methods well-known and developed to a high degree in the dialysis arts. Patient blood, as it flows to the apparatus I0 (alternately referred to herein as puvapheresis apparatus or system) is preferably infused, under control of pump II, with an anti-coagulant agent contained in container 20 hung from stand I5. Control of the flow of patient blood to the remainder of apparatus I0 is controlled largely by clamping means I6a which has the dual function of also controlling flow in the reverse direction as well as flow to return container 2I. Clamp I6a acts as an "or" valve.

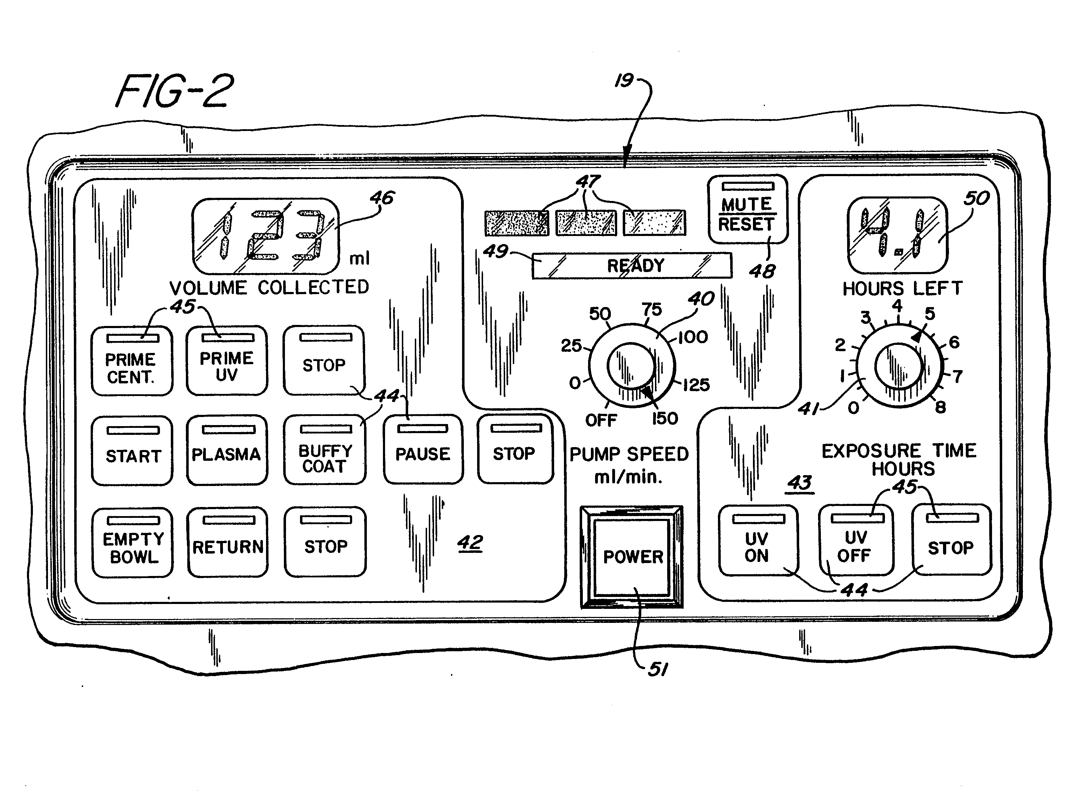

Normally the blood flows through tubing 24 through blood pump I2 (preferably a roller pump such as that described in U.S. Patent No. 4,487,558 to Troutner entitled "Improved Peristaltic Pump" and incorporated herein by reference) into continuous centrifuge I3. This continuous centrifuge, available commercially from suppliers such as Dideco, Haemonetics and others, is preferably capable of continuously separating blood based on the differing densities of the individual blood components. "Continuously", as used herein means that, as blood flows into the centrifuge through line 24, it accumulates within the rotating centrifuge bowl and is separated so that low density components are emitted after a certain minimum volume has been reached within the centrifuge bowl and as additional blood is added. Thus, the continuous centrifuge in effect acts as a hybrid between a pure online system and a pure batch system. This occurs because the centrifuge bowl has a capacity to hold most, if not all, of the most dense portion, typically erythrocytes or red blood cells while emitting lower density portions such as plasma and leukocytes (white blood cells) as whole blood is continuously added. At some point, however, the reservoir volume of the centrifuge is filled with the higher density components and further separation cannot be effectively obtained. Prior to that point, the operator, by viewing the uppermost portion of the centrifuge bowl through the centrifuge cover, can detect qualitatively when the centrifuge emits plasma (as opposed to priming solution), leukocyte enriched portions and the remainder, i.e., nonleukocyte enriched portions, including erythrocyte enriched portions. Based on the operator's observations, he or she enters through control panel I9 (specifically via panel portion 42) the identification of the individual blood portions as they are emitted from the centrifuge. This information is entered by keys 44 (e.g. PLASMA, BUFFY COAT or leukocyte enriched portion) on control panel I9, (shown in Figure 2) and in response thereto, the apparatus I0 controls valve mechanism I6c to direct the leukocyte enriched portion and a predetermined volume of plasma into plasma-leukocyte enriched container 22 while excess plasma, air, priming fluids, erythrocytes etc. are directed to container 2I.

Once the centrifuge is no longer capable of further separation due to the attainment of its capacity, the operator directs that the bowl be emptied by suitable data key entry on panel I9 and the fluid contents of centrifuge I3 are advantageously pumped into return container 2I by means of pump I2 under the control of valves I6a and c. The foregoing steps may be repeated a number of times or cycles before the desired volume of leukocyte enriched blood and plasma is obtained for further treament, in each instance the undesired portions being collected in return container 2I.

Between cycles, the fluids, including erythrocytes which have been pumped into return bag 21 are gravity fed back to the patient through a drip infusion operation and controlled by valve 16b. It is preferred that gravity feed be employed rather than pumping the blood back to the patient via pump 12 in order to avoid potential pressurization problems at the infusion insertion site at the patient, and also to avoid foaming or other air related dangers.

As may be already appreciated, when initially set up, the centrifuge bowl and line 24 may be expected to contain sterilized air which is preferably removed by suitable priming operations advantageously accomplished by utilizing the anticoagulation agent in container 20; both the air and a portion of priming solution being collected in container 21.

Also to be noted is the predetermination of the desired leukocyte enriched volumes and plasma volume to be collected within container 22 as well as the number of cycles to be employed to collect same. These volumes are selected largely in accordance with the individual volume capacities of the containers as well as the treatment cassette to be described later. Accordingly, these volumes are set in order to preferably optimize handling efficiency and to ensure patient safety. For instance, one preferred selection would include the following settings: 250 ml total buffy coat or leukocyte enriched portion and 300 ml of plasma to be collected within container 22. This might require any number of cycles, preferably on the order of three or four, bearing in mind that the more cycles that are selected, the lower the total volume of blood withdrawn from the patient at any one time. If blood collection meets the minimum capacity limits of the centrifuge bowl, the patient's capacity to withstand temporary blood volume depletions, and the treatment procedure in general is increased. Further, more cycles will permit more discriminating selection of leukocyte enriched blood as it is emitted from the centrifuge. The buffy coat and plasma volumes as well as the number of cycles are typically physician selected.

Accordingly, the controls governing these selections are preferably placed within the apparatus 10, such as behind door 18a where their inadvertent alteration may be advantageously avoided, especially since no operator interaction is normally required with respect to these data inputs.

The leukocyte enriched container 22 is connected via tubing line 34 to the flat plate treatment cassette behind cassette assembly door 17 with a return line 35 to reservoir container 22.

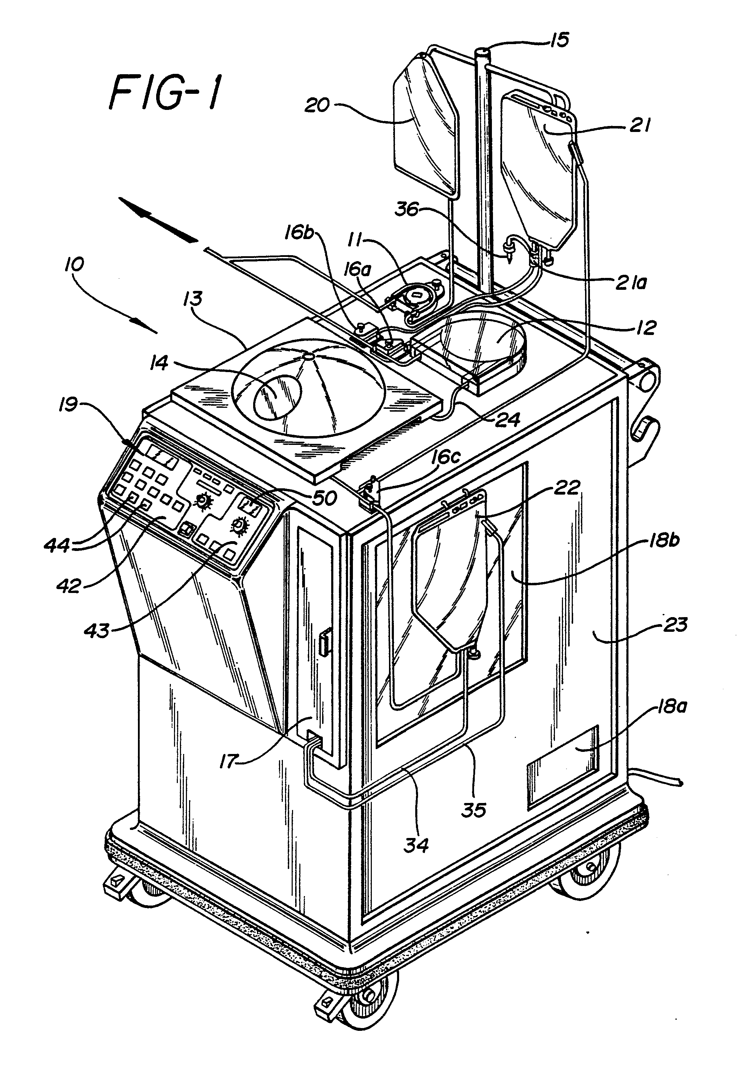

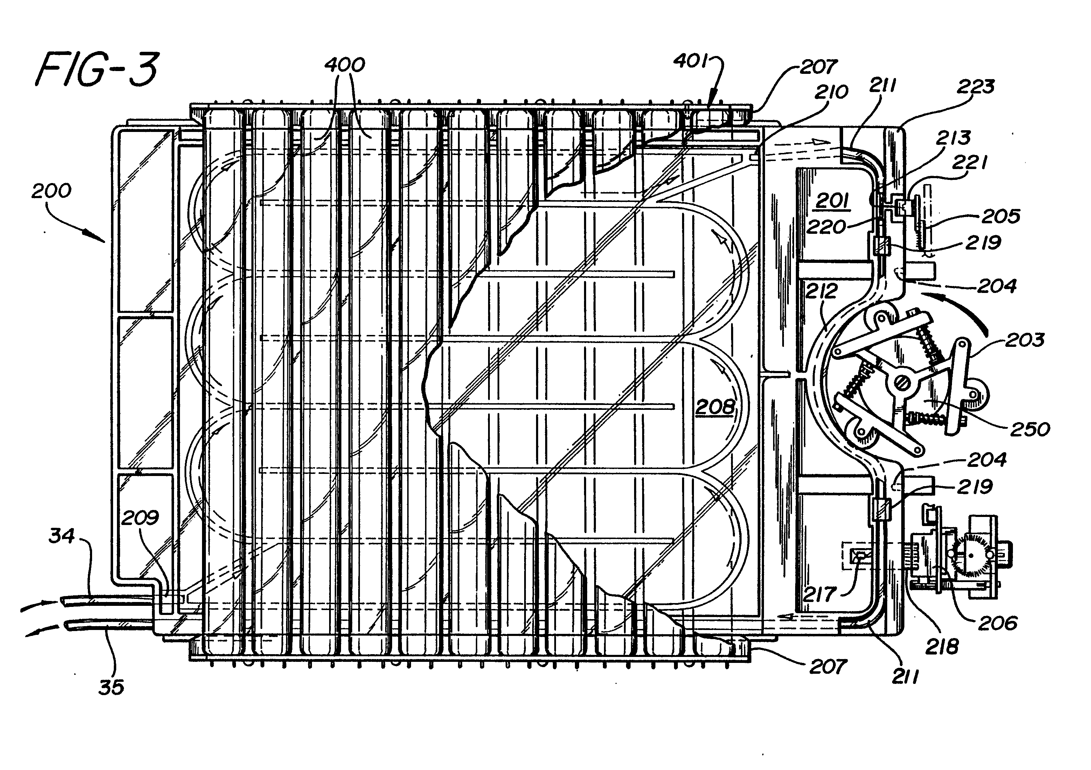

Referring now to Figure 3, the leukocyte enriched blood, plasma, and priming solution contained in reservoir 22 (Figure 1) is delivered through line 34 to the inlet 209 of the flat plate irradiator 200. The cavity in the flat plate is relatively "thin" (e.g. on the order of approximately 0.04 inch) in order to present large surface area of leukocyte rich blood to irradiation and reduce the self-shielding effects encountered with lower surface area/volume ratios. The fluid flows upward through the serpentine pathway in cavity 208 in the irradiation chamber to the outlet 210. While a serpentine pathway is preferred in order to avoid or minimize stagnant areas of flow, other arrangements are contemplated. Tubing from the outlet 211 passes through the pump block 201 [described in greater detail in EP-A-     (JJC-7)], affixed to the end of the flat plate irradiator 200, and then connects to return line 35 which returns fluids from the irradiation chamber to container 22.

Recirculation pump rotor 203, which is located internally in the machine (mounting not shown), engages the tubing in the pump block in the semi-circular tract 212 and thereby provides and controls the recirculating flow of fluid, from container 22 up through irradiation chamber 200 and back to container 22. In a preferred embodiment, a metal segment 220 in the tubing line from outlet 211 incorporates a thermocouple 213 [described more fully in EP-A-     (JJC-4)] which permits monitoring of the fluid temperature.

Sterile air initially contained in the irradiation chamber cavity 208 is displaced by entering fluid and stored in the top of container 22. By reversing the rotation of recirculation pump rotor 203, the air stored in container 22 can be pumped back into the outlet 210 of the chamber 200 thereby displacing all fluids back into container 22. Once fluid is initially delivered to container 22, the recirculation pump rotor 203 is energized filling the irradiation cavity 208 and displacing sterile air to container 22. When the irradiation chamber is filled and BUFFY COAT button 44 on panel 19 is pressed, the light array assembly which surrounds the irradiation chamber is energized. Continued operation of the recirculation pump rotor 203 continuously recirculates the leukocyte enriched fluid from container 22 through the chamber for receiving photoactivating radiation from the energized light array assembly 401 (Figure 4) and back to container 22.

Figure 4, illustrating the light array assembly 401 from a bottom view, shows two rows, in the most preferred embodiment although one row can be used, of radiation source 400 powered through contacts 216. Such sources are conveniently chosen so that illumination is reasonably constant over the entire irradiation cavity 208 (Figure 3). Suitable sources include the Sylvania FR15"T8/350BL/HO/180° with 2011

phosphorus bulb which is in the so-called fluorescent tube form. As is apparent from Figure 4, the irradiation chamber 200 slides between the rows of radiation source 400 so that pump block 201 engages pump rotor 203 driven by motor 250. Other aspects of the radiation array 400 are discussed in EP-A-(JJC-6).

Thus, photoactivation of the leukocyte enriched fluid by irradiation is initiated at the outset and continues through and after the collection and separation process. In the most preferred mode, the light array assembly [described more fully in EP-A- (JJC-6)] will comprise sources of ultraviolet radiation, most preferably of the UVA type for activating the photoactivatable agent presently of choice, 8-methoxy psoralen.

The flat plate irradiation chamber treatment module is described more fully in EP-A- (JJC 14).

In operation, and with respect to Figure 2, the exposure time on the right hand portion of the panel 43 is set in accordance with physician determined criteria via knob 4l. The central control means of the apparatus l0, calculates and displays (50) via central processing unit and memory stored software, the exposure time remaining at the onset of irradiation treatment and as the treatment progresses. Section 43 of the control panel also includes three operator controlled entry data keys 44 whereby the operator can de-energize the photoactivating light array and stop the recirculation process if desired. Actual photoirradiation treatment preferably commences automatically under control of the central processing unit when fluid is first directed to container 22, continues while leukocyte enriched blood portion from container 22 is pumped through the irradiation chamber back into container 22, and terminates when the preset exposure time has expired. At that time, the light array assembly is de-energized and the recirculation pump reverses emptying the contents of the irradiation chamber 200 into container 22.

Thereafter container 22 is ideally removed to stand l5 (Figure l) where it is connected to tube 36, provided on the common drip chamber 2la also associated with return container 2l, for reinfusion of the treated blood portion into the patient.

To enhance patient safety and decrease the risk of contamination to the patient blood and blood portions, each time a connection is made or broken, it is preferably only done once. Thus, container 22 would ideally have four connection points or ports; one for the collection of the leukocyte enriched blood portion, two for connection to the flat plate irradiation cassette (feed and return), and the fourth for connection to the drip chamber (2la) for reinfusion of treated blood to the patient.

With further reference to Figures 2 and 3, the control panel l9 of the apparatus l0 is shown with the keyboard entry buttons 44, each ideally having a light 45 which, when lit, preferably indicates the stage of the operation. As will be noted, the keyboard entry buttons 44 are preferably placed in sequential order thereby assisting the operator in learning the system and performing the steps in the correct order. Indeed, the central control microprocessor will preferably be programmed to prevent out of step sequences from being implemented. The entry buttons may also be optionally equipped with tactile and/or auditory feedback ques in place of or in addition to the lights for operator convenience. A visual display 46 indicates the volume of leukocyte enriched blood collected in container 22.

Panel l9 will preferably also contain a power switch 5l, as well as a blood pump speed control 40 whereby the operator may select the speed with which the blood is withdrawn from the patient and pumped through the system during collection. Also preferably included is an alpha-numeric display 49 for indicating the machine's status and identifying alarm conditions throughout system operation. Optional accessory status lights 47, preferably provided in green, yellow, and red colors, provide at a glance the overall operating status of apparatus l0. Further included is a mute/reset button 48 for quieting an audible alarm activated in the event an alarm condition occurs and operator input is required.

Other features may be readily apparent from the drawings such as the preferable inclusion of casters and caster brakes for enhancing the mobility of the apparatus. Further, side panel 23 will preferably include mechanical means (e.g. hanging pegs and the like) for assisting in the securement of container 22. It may also optionally be outfitted with a transparent or translucent opening l8b in the area beneath container 22 for providing at a glance information regarding the illumination status of the irradiation treatment cassette during the treatment phase. For instance, if the window is of sufficient size, the operator may readily determine that each irradiation source within the treatment cassette is illuminated as desired. Naturally, the material comprising such window is preferably selected in order to contain harmful radiation, if any, within apparatus l0.

The aforedescribed photopheresis blood treatment apparatus is made largely possible by an automated control method for directing the blood portions, derived from the continuous centrifuge, into particular containers. The automated method performs in accordance with preset volume determinations which are manually entered behind panel l8a pursuant to a physician's direction. These predetermined volumes

6

specify the volume to be contained within container 22 by setting forth the volume of plasma and the volume of leukocyte enriched blood portion to be directed thereto. Additionally included within these condition setting parameters is preferably the ability to set forth the number of cycles of blood collection and separation required or desired in order to obtain the desired blood volumes.

The volumes collected are determined in accordance with the blood volume pumped by the blood pump. This may be suitably monitored and communicated to the central control means by specifically monitoring the number of step pulses input to the pump to cause rotations of the blood pump. Typically, 200 pulses results in one revolution. Rotation may also be conveniently monitored such as by attachment of a slotted disk to the shaft and the passage of slots determined by an optical sensor means such as that described in US-A-4 623 328 and by monitoring shaft rotation. The resultant periodic signal may be conveniently correlated with speed and number of rotations by circuit designs well-known in the art. The number of rotations by any of the foregoing methods coupled with the known volume pumping characteristics of the pump, will provide the necessary information regarding the volume of blood pumped. It will readily be appreciated that the sensors need not be optical but may be electronic or mechanical instead.

In actual operation, a most preferred procedure would be as follows. The operator presses the PRIME CENT. key on control panel section l9 which primes the tubing set, the blood pump, and the centrifuge with the anti-coagulation solution contained in container 20. Displaced sterile air is collected in container 2l. When priming solution emerges from the exit of the centrifuge, the operator presses PRIME UV key on control panel section 42 which closes the tubing line to container 2l and opens the tubing line to container 22 by means of valve l6c. Recirculation roller pump rotor 203 is energized to prime the flat plate irradiation chamber and displace sterile air to container 22. The priming process stops automatically after a preset volume of fluid is delivered to container 22.

Collection is started by the operator pressing START key on control panel l9. Thereafter, blood is withdrawn from the patient and pumped by the blood pump into the rotating centrifuge. As the blood enters the centrifuge, it displaces the priming solution which emerges first in accordance with its preferably lighter density. This priming solution is automatically directed into container 22 until a preset volume is delivered, after which the emerging solution is redirected to container 2l by means of valve l6c. At some point, the priming solution will be completely displaced from the rotating centrifuge and plasma will begin to emerge. This emergence may be directly observed through port l4 whereupon the operator presses the PLASMA key on control panel l9. Thereafter, the central control means automatically directs the plasma into container 22 by altering valve l6c keeping track of the volume as it does so since the volume entering the centrifuge equals the volume emerging therefrom. This continues until the operator indicates the leukocyte enriched portion, i.e. buffy coat has begun by pressing the respective data entry key in control panel section 42 whereupon, the leukocyte enriched portion continues to container 22, however, the volume so directed is monitored as buffy coat volume. Alternately, if all of the predetermined plasma volume is collected prior to the emergence of the buffy coat, then the central control means automatically diverts, by valve l6c, the emerging plasma fluid stream to container 2l. In that instance, upon the emergence of the buffy coat and the keying of the BUFFY COAT data entry switch 44, the central control means diverts the emerging buffy coat into container 22, by means of valve l6c, again keeping track of its volume.

The collection of the buffy coat will preferably continue in accordance with both the predetermined buffy coat volume as well as the number of cycles, another condition predetermined by the physician. If this most preferred embodiment is employed, then a representative example might be as follows. Assume, that the predetermined volume and cycle conditions are set as follows: 350 mls of plasma, 250 mls of buffy coat, and 5 cycles. In each cycle, the apparatus will collect 250/5 or 50 mls of buffy coat before ending the cycle and thereupon emptying the centrifuge bowl and returning all nonleukocyte fluids, predominantly erythrocytes and perhaps excess plasma, to the patient. Prior to the collection of the 50 mls, plasma will emerge from the centrifuge and will be collected in container 22 either until the full 350 mls are collected or, until the buffy coat emerges.

During the next cycle, the central control means will direct the further collection of plasma, if needed, in order to reach the 350 ml predetermined volume and then collect an additional 50 mls of buffy coat. The total volume to be contained within container 22, will then equal 600 mls and would be indicated on display 46 as it is accumulated.

Thus, the instant invention serves to automatically keep track of the volumes as they are collected thereby facilitating the institution of a convenient number of cycles whereby the removal of large blood volumes from the patient is avoided. Not only is patient safety enhanced thereby, but the automated nature of the procedure further increases safety since, in accordance with the programmed conditions supplied to the central control microprocessor or computer, the operator need not attempt to keep track of plasma and leukocyte enriched volumes collected, while still being assured that the final solution for treatment will contain the predetermined and desirable leukocyte concentration. The most preferred apparatus will also contain a memory feature associated with the central control or processing unit which remembers the exact point during the operation the machine was performing in the event of a power failure occasioned by time power failure or inadvertent power cord disconnection. Thus, upon repowering the apparatus, the operator may direct the treatment operation to recommence as if the interruptions had not taken place.

Efficiency of operation can be maximized by concurrently performing the steps of collecting blood from the patient, separating it into the desired leukocyte enriched portion and plasma, irradiating the leukocyte enriched portion and plasma while reinfusing the patient with the remaining blood portions, essentially erythrocyte enriched, and excess plasma, if any, obtained from the separation processes. The patient remains connected to the apparatus until irradiation is completed at which time the patient is reinfused with the irradiated leukocyte enriched blood portion.

By concurrently performing the collection, separation, irradiation, and re-infusion, a number of unobvious advantages are gained. Starting the irradiation soon after collection begins, coupled with the large irradiation area offered by the flat plate irradiation chamber configuration, results in a substantial reduction of total treatment time. Thus, a complete treatment which previously required a full 8 hour day using the prior sequential methods, can now be accomplished in less than 4 hours. This makes it possible to perform two treatments with a single machine in a clinical 8 hour day. Other unobvious advantages gained are that by reducing treatment time, the patient can remain connected, or on-line, during the entire treatment thus making a second venapuncture for re-infusion unnecessary. This not only increases patient comfort, but also reduces patient trauma. A major safety advantage is also obtained in that by remaining connected throughout treatment, the possibility of re-infusing blood from a different patient is eliminated. This latter possibility represents a substantial hazard in a busy, multi-patient clinical situation where patients are disconnected during irradiation and reconnected for re-infusion.

Still another problem is solved by the instant invention and this concerns the development of treatment systems, apparatus and methods which may be readily understood, controlled and performed by technicians who do not possess advanced degrees or require other detailed instruction. By concurrently performing all operations under the control of the central processing unit or control means, the technician is more readily capable of grasping the sequence of steps and accomplishing the desired irradiation treatment safely, efficaciously, and rapidly. Thus, the complement of personnel who may be trained to perform photoactivation patient treatment procedures is expanded. It is also hoped that this will further enhance the acceptability of the Edelson procedures within the clinical environments thereby benefiting more patients.

Finally, the concurrent process of the instant invention facilitates the design and manufacture of irradiation chamber 200, thus reducing costs, an important criteria in view of recent fiscal constraints placed on the clinical environment.

From the foregoing description, one of ordinary skill will readily appreciate that numerous insignificant changes regarding procedural details, mechanical variations in design, and the like may be made without departing from either the spirit or scope of the instant invention.

## Claims

I. A system for concurrently altering blood cells from a patient by treating the cells with a photoactivatable agent, irradiating the cells and the agent to activate the agent so that the agent affects the cells, and returning the cells to the patient, the system comprising:

(a) means for collecting whole blood in contact with a pharmaceutically effective amount of the photoactivatable agent from a patient;

(b) means for passing the whole blood to a continuous centrifuge adapted to produce plasma and a leukocyte enriched portion;

(c) means for collecting from the centrifuge a predetermined volume of plasma and leukocyte enriched portion;

(d) means for returning non-leukocyte enriched portions and any excess plasma to the patient;

(e) means for concurrently passing the collected plasma and leukocyte enriched portion through an irradiation chamber adapted to photoactivate the agent until a predetermined level of photoactivation has been achieved; and

(f) means for returning the irradiated plasma and leukocyte enriched portion to the patient without passing said irradiated plasma and leukocyte enriched portion through said continuous centrifuge, the system being adapted to be operated so that the patient can remain connected to the system until the irradiation is completed and the irradiated leukocyte enriched portion is returned to the patient.

2. The system of claim I, further including means for infusing anticoagulation means into the whole blood as it is collected.

3. The system of claim I or claim 2, which is adapted to operate means (a), (b) (c) and (d) a plurality of times in sequence substantially before means (e) and (f) are operated in sequence.

4. The system of any one of claims I to 3, further including means for collecting the non-leukocyte enriched portions and any excess plasma separately from the leukocyte enriched portion and plasma.

5. The system of claim 4, wherein the means for collecting the non-leukocyte enriched portion and any excess plasma comprises an elevated container adapted for gravity infusion back to the patient.

6. A system for altering cells collected from a patient wherein said cells are in contact with a pharmaceutically effective amount of a photoactivatable agent, said cells and agent are irradiated for photoactivating the agent, and said irradiated cells are returned to said patient, comprising:

(a) means for collecting said cells from a patient;

(b) continuous centrifuge means for obtaining a cell enriched portion and a non-cell enriched portion;

(c) means for returning to said patient said non-cell enriched portion;

(d) pump means for passing said cell enriched portion through irradiation chamber means for exposing said cell enriched portion to activating irradiation, said pump being capable of operating concurrently while said means (c) is operating;

(e) irradiation means associated with said irradiation chamber for providing said activating irradiation; and

(f) means for collecting said irradiated cells and returning same to said patient without passing said cells through said continuous centrifuge means (b).

7. In a system for altering blood cells from a patient including treating the cells with a photoactavatible agent and irradiating said cells and said agent whereby said agent is caused to be activated and to affect said cells, a concurrent method for so treating the blood cells of a patient comprising the steps of:

(a) collecting whole blood from a patient, said blood being in contact with a pharmaceutically effective amount of a photoactivatable agent;

(b) passing said whole blood into a continuous centrifuge for obtaining plasma and a leukocyte enriched portion;

(c) collecting from said centrifuge a predetermined volume of said plasma and said leukocyte enriched portion;

(d) returning non-leukocyte enriched portions and any excess plasma to said patient;

(e) concurrently with step (d), passing said collected plasma and leukocyte enriched portion through an irradiation chamber for photoactivating said agent, until a predetermined level of photoactivation has been achieved; and

(f) returning said irradiated plasma and leukocyte enriched portion to said patient without passing said irradiated plasma and leukocyte enriched portion through said continuous centrifuge, wherein the patient remains connected to said treatment system until said irradiation is completed and said irradiated leukocyte enriched portion is returned to said patient.

FIG-1

FIG-2

VOLUME COLLECTED — *123* ml (46)

PRIME CENT. (45) | PRIME UV | STOP
START | PLASMA | BUFFY COAT (44) | PAUSE | STOP
EMPTY BOWL | RETURN | STOP

*42*

47 | MUTE RESET
49 — READY | 48
19

PUMP SPEED ml/min.
OFF | 0 | 25 | 50 | 75 | 100 | 125 | 150 | 40

POWER (51)

HOURS LEFT — *4.1* (50)
0 1 2 3 4 5 6 7 8 (41)

EXPOSURE TIME HOURS
*43* | *45*
UV ON | UV OFF | STOP
44

FIG-3

FIG-4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A-2 100 143 (EDELSON)<br>* claim 1; abstract; figure 1 *<br><br>--- | 1,6,7 | A 61 M 1/36 |
| P,A | US-A-4 573 960 (GOSS)<br>* claim 1; column 5, lines 21-35; figure 1 *<br><br>----- | 1,4-7 | |

TECHNICAL FIELDS
SEARCHED (Int. Cl.4)

A 61 M 1/00

The present search report has been drawn up for all claims

| Place of search | Date of completion of the search | Examiner |
|---|---|---|
| BERLIN | 13-07-1987 | PAPA E.R: |